# EUROPÄISCHE PATENTSCHRIFT

(11) **EP 0 716 712 B1**
(45) Veröffentlichungstag und Bekanntmachung des Hinweises auf die Patenterteilung: **04.11.1998**
(21) Anmeldenummer: 94926853.6
(22) Anmeldetag: 19.08.1994
(51) Int. Cl.: C12P 7/02, C12P 41/00

(54) **LIPASE KATALYSIERTE ACYLIERUNG VON ALKOHOLEN MIT DIKETENEN**
LIPASE-CATALYSED ACYLATION OF ALCOHOLS WITH DIKETENES
ACYLATION D'ALCOOLS AVEC DES DICETENES CATALYSEE PAR DES LIPASES

(30) Priorität: 31.08.1993 DE 4329293
(43) Veröffentlichungstag der Anmeldung: 19.06.1996
(73) Patentinhaber: BASF AKTIENGESELLSCHAFT, 67056 Ludwigshafen (DE)
(72) Erfinder: BALKENHOHL, Friedhelm, D-67117 Limburgerhof (DE); HAUER, Bernhard, D-67136 Fussgoenheim (DE); LADNER, Wolfgang, D-67136 Fussgoenheim (DE); SCHNELL, Ursula, D-67136 Fussgoenheim (DE); PRESSLER, Uwe, D-67122 Altrip (DE); STAUDENMAIER, Horst Ralf, D-67134 Birkenheide (DE)
(86) Internationale Anmeldenummer: EP9402757
(87) Internationale Veröffentlichungsnummer: WO9506746

(56) Entgegenhaltungen:
- EP-A- 0 321 918
- EP-A- 0 357 009
- EP-A- 0 492 497
- DE-A- 4 100 394
- SYNTHESIS, vol., no., 1992, page 895 - 910, K. FABER UND S. RIGA: 'ENZYME-CATALYZED IRREVERSIBLE ENZYME TRANSFER.'

## Beschreibung

Die vorliegende Erfindung betrifft ein Verfahren zur Herstellung von acylierten Alkoholen aus Alkoholen, insbesondere zur Herstellung von enantioselektiv acylierten Alkoholen aus racemischen Alkoholen.

Die Herstellung von acetoacetylierten Alkoholen durch Umsatz von Alkohol mit Diketen ist bekannt. Diese Reaktion wird in der Regel unter Säure- oder Basenkatalyse unter erhöhten Temperaturen durchgeführt. Für empfindliche, leicht umlagernde Alkohole sind diese Bedingungen der Acetoacetylierung nicht geeignet.

Bekannt ist ein Verfahren zur Racematspaltung von racemischen Alkoholen mit Vinylestern in Gegenwart von Lipasen als Katalysator (EP 321 918). Als geeignete Acylierungsmittel werden hier Vinylester der allgemeinen Formel I 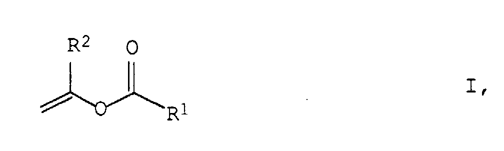 wobei
R¹ Wasserstoff, Alkyl, Phenyl oder Alkoxyalkyl und
R² Wasserstoff oder Methyl bedeutet, beschrieben.

Bei der Umesterung entstehen aus den Vinylestern I die Nebenprodukte Acetaldehyd bzw. Aceton, die abgetrennt werden müssen, da sie Enzyme inaktivieren können. Weiterhin haben bei Verwendung von Vinylacetat als Acylierungsmittel Alkohol und Ester sehr nahe beieinanderliegende Siedepunkte, so daß sie sich nur mit großem Aufwand destillativ trennen lassen.

In einem Übersichtsartikel zu enzymkatalysierten Acyl-Übertragungen (Faber and Riva, Synthesis 895-910, 1992) wurde beschrieben, daß das Acylierungsmittel Diketen für enzymatische enantioselektive Acetoacetylierungen nicht geeignet ist, da es nur zu einem äußerst geringen Enantiomerenüberschuß führt.

Die beiden racemischen Alkohole II und III wurden mit Diketen in Toluol bei Raumtemperatur in Gegenwart von Candida cylindracea Lipase zu den entsprechend acetoacetylierten Verbindungen IIa bzw. IIIa umgesetzt. Diese Reaktion wies jedoch keine Enantioselektivität auf, wie sich aus dem Enantiomerenüberschuß von weniger als 10 % ablesen läßt (o.a. S. 900).

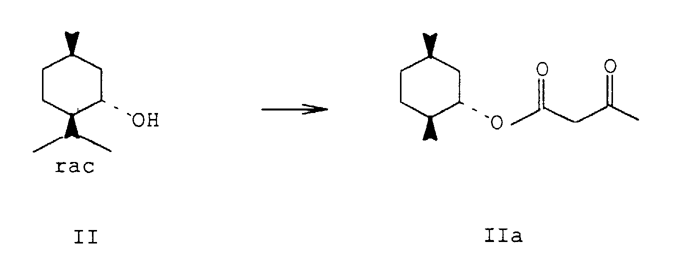

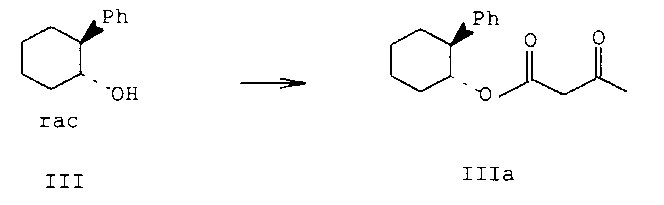

Weiterhin wurde dort festgestellt, daß keine spezifische Lipase-Katalyse mit Diketen stattfand, sondern eine unspezifische Basenkatalyse durch Protein, die auch mit Rinderserum Albumin - das keine Esteraseaktivität aufweist - möglich war.

Aufgabe der vorliegenden Erfindung war es, ein Verfahren zur Herstellung von acylierten Alkoholen aus Alkoholen bereitzustellen, das die Veresterung des Alkohols unter möglichst schonenden Bedingungen erlaubt.

Eine weitere Aufgabe war es, ein Verfahren bereitzustellen, das es gestattet, selektiv optisch aktive Alkohole aus racemischen Alkoholen darzustellen, ohne daß die oben beschriebenen Nachteile auftreten.

Demgemäß wurde gefunden, daß sich Alkohole mit einem Diketen zu acylierten Alkoholen unter besonders milden Bedingungen umsetzen lassen, wenn die Umsetzung unter spezifischer Lipase-Katalyse durchgeführt wird.

Weiter wurde gefunden, daß sich enantioselektiv acylierte Alkohole aus racemischen Alkoholen darstellen lassen, wenn man die racemischen Alkohole mit einem Diketen in Gegenwart von Lipase umsetzt, d.h. die Reaktion unter spezifischer Lipase-Katalyse durchführt.

Weiterhin wurde ein Verfahren zur Herstellung von optisch aktiven Alkoholen aus racemischen Alkoholen, dadurch gekennzeichnet, daß man
a) einen racemischen Alkohol mit einem Diketen unter spezifischer Lipasekatalyse enantioselektiv acyliert,
b) das Gemisch aus optisch aktivem Alkohol und optisch aktivem acyliertem Alkohol trennt und somit ein Enantiomer des Alkohols erhält,
c) gewünschtenfalls aus dem acylierten Alkohol, das andere Enantiomere des Alkohols durch Esterspaltung gewinnt,
gefunden.

Diese erfindungsgemäßen Verfahren eignen sich besonders zur Herstellung von optisch aktiven Verbindungen.

Die Acylierung von Alkoholen geschieht nach folgender Reaktionsgleichung:

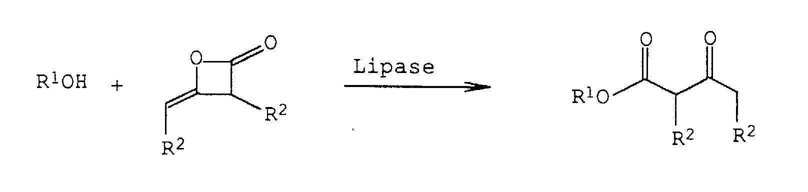

Die als Ausgangsstoffe dienenden Alkohole R¹OH können primäre, sekundäre oder tertiäre Alkohole sein. Geeignet für das Verfahren sind sowohl Alkyl- als auch Arylalkylalkohole.

Als Diketen kann sowohl das Dimerisationsprodukt des Ketens CH₂=C=O als auch eines substituierten Ketens R²HC=C=O verwendet werden. Der Rest R² kann einen Alkyl- oder einen Arylrest bedeuten.

Bevorzugt wird als Diketen das Dimere des unsubstituierten Ketens (R² = Wasserstoff) verwendet. In diesem Fall führt die Acylierung zu acetoacetylierten Alkoholen.

Bei der Verwendung von racemischen Alkoholen findet unter den beschriebenen Bedingungen eine enantioselektive Acylierung statt. Das eine Enantiomer des Alkohols wird acyliert, während das andere Enantiomer des Alkohols unverändert bleibt. Diese selektive Reaktion erlaubt die Auftrennung von racemischen Alkoholen in ihre Enantiomere.

Das Diketen wird in der Regel in einem leichten molaren Überschuß, bezogen auf den umzusetzenden Alkohol, eingesetzt. Bevorzugt werden pro Mol Alkohol 1,1 Mol Diketen verwendet. Für die Umsetzung von Diketen mit racemischen Alkoholen bedeutet das, daß pro Mol Racemat 0,5 bis 0,55 Mol Diketen eingesetzt werden, da nur ein Enantiomer mit Diketen unter den angegebenen Bedingungen reagiert.

Als Lösungsmittel sind generell organische Lösungsmittel geeignet. Besonders gut verläuft die Reaktion in Ethern, beispielsweise in MTBE oder THF, oder in Kohlenwasserstoffen wie Hexan, Cyclohexan, Toluol oder halogenierten Kohlenwasserstoffen wie Methylenchlorid.

Als für das Verfahren gut geeignete Katalysatoren haben sich bakterielle Lipasen herausgestellt. Besonders geeignet sind Lipasen aus Pseudomonas, beispielsweise die Lipase Amano P® oder die Lipase aus Pseudomonas spec. DSM 8246.

Diese Lipase hat sich als besonders aktiv, selektiv und resistent hinsichtlich einer Inaktivierung erwiesen.

Es sind jedoch auch eine Reihe anderer Lipasen, beispielsweise Candida cylindraceae Lipase (CCL) für das erfindungsgemäße Verfahren geeignet.

Die Lipase kann auch an einen unlöslichen Träger adsorptiv oder kovalent gebunden eingesetzt werden.

Die Umsetzung des Alkohols mit dem Diketen erfolgt in der Regel bei Raumtemperatur.

Nach Beendigung der Reaktion wird im allgemeinen die Lipase abfiltriert, das Lösungsmittel und das überschüßige Diketen aus dem Filtrat entfernt und das Gemisch aus acyliertem Enantiomer und nicht umgesetztem Enantiomer mit üblichen Methoden aufgetrennt. War das gewünschte Enantiomer der nicht umgesetzte Alkohol, so liegt es nun bereits in reiner Form vor; war das acylierte Enantiomer das gewünschte Enantiomer, so muß es noch durch eine übliche Esterspaltung aus dem Acetessigester freigesetzt werden.

Das erfindungsgemäße Verfahren wird in der Regel so durchgeführt, daß die Ausgangsverbindungen Alkohol und Diketen im Lösungsmittel vorgelegt werden und die Reaktion durch Zugabe der Lipase gestartet wird.

Die Lipase kann gewünschtenfalls nach Beendigung der Reaktion zurückgewonnen und für eine neue Umsetzung wiederverwendet werden.

Das erfindungsgemäße Verfahren hat den Vorteil, daß es unter einfachen und sehr milden Bedingungen verläuft. Es ist daher auch gut für empfindliche sowie S_{N}1-aktive Alkohole (z.B. 1-Phenylethanol) geeignet. Es entstehen dabei keine Nebenprodukte, die die Lipase hemmen können. Weiterhin sind die entstandenen Produkte aufgrund ihrer unterschiedlichen Siedepunkte gut destillativ trennbar.

Die folgenden Beispiele dienen der Veranschaulichung der Erfindung.

### Beispiel 1

### Allgemeine Vorschrift zur Herstellung von Acetessigestern

55 mmol Diketen und 50 mmol Alkohol wurden in 30 ml THF gelöst. Nach Zugabe von 50 mg Lipase (Pseudomonas spec., 658 U/mg) wurde bei Raumtemperatur gerührt. Nach vollständigem Umsatz (4 h) wurde die Lösung filtriert und im Vakuum aufkonzentriert (50°C, 30 mbar). Man erhielt den NMR-reinen Acetessigester in einer Ausbeute von über 90 %.

Nach dieser Vorschrift wurden Ethanol, Isopropylalkohol und Benzylalkohol umgesetzt.

### Beispiel 2

### Kinetische Racematspaltung von 1-Phenylethanol:

55 mmol Diketen und 50 mmol Phenylethanol (rac.) wurden in 30 ml THF gelöst. Die Reaktion wurde durch Zusatz von 100 mg Pseudomonas spec. DSM 8246 (siehe Beispiel 1) gestartet. Per GC wurde der Umsatz der Reaktion kontrolliert. Simultan wurde der Drehwert der filtrierten Reaktionslösung verfolgt. Bei einem Umsatz von ca. 50 % (Drehwertmaximum, ca. 5 h) wurde die Reaktion durch Abfiltrieren der Lipase abgebrochen. Das Produktgemisch wurde eingeengt und durch Chromatographie an Kieselgel (Cyclohexan: Essigester = 5:1) aufgetrennt.

Man erhielt:
- 5,3 g: R-(+)-1-Phenylethanol-acetoacetat (52 % Ausbeute)
ee = 75 % (87,5:12,5)
[α]_{D}²⁰ = +84,5° (c = 1,078 in Toluol)
- 2,1 g: S-(-)-1-Phenylethanol (34 % Ausbeute)
ee = 97 % (98,5:1,5)
[α]_{D}²⁰ = -43,8° (c = 0,984 in Toluol)

Die Enantiomerenüberschüsse wurden durch 500 MHz ¹H-NMR der Mosher-Ester bestimmt (JACS 95 (1973) 512):

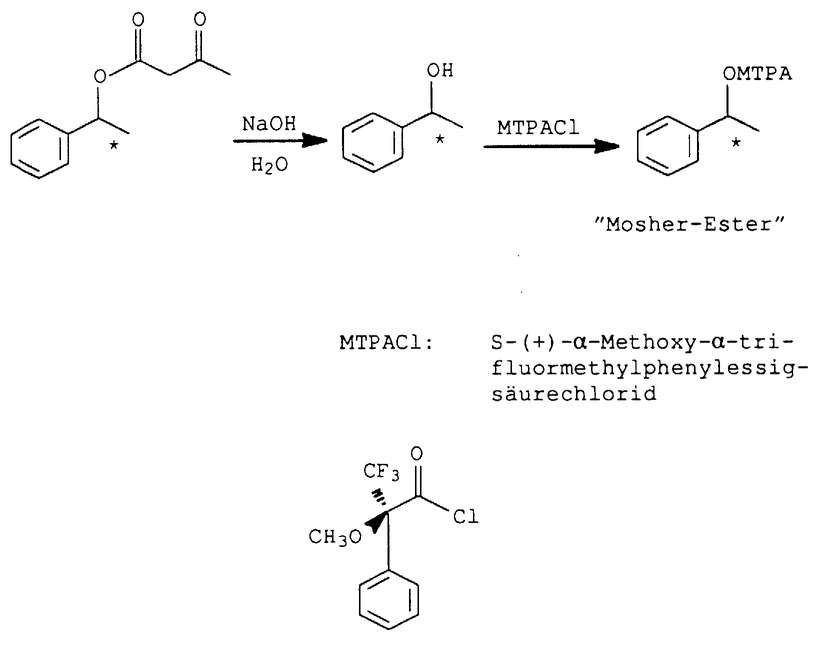

## Patentansprüche

1. Verfahren zur Herstellung von enantioselektiv acylierten Alkoholen aus racemischen Alkoholen, dadurch gekennzeichnet, daß man einen racemischen Alkohol mit einem Diketen unter spezifischer Lipase-Katalyse umsetzt.

2. Verfahren zur Herstellung von optisch aktiven Alkoholen aus racemischen Alkoholen, dadurch gekennzeichnet, daß man
a) einen racemischen Alkohol mit einem Diketen unter spezifischer Lipase-Katalyse enantioselektiv acyliert,
b) das Gemisch aus optisch aktivem Alkohol und optisch aktivem acyliertem Alkohol trennt und somit ein Enantiomer des Alkohols erhält,
c) gewünschtenfalls aus dem acylierten Alkohol, das andere Enantiomere des Alkohols durch Esterspaltung gewinnt.

3. Verwendung eines Verfahrens gemäß Anspruch 1 oder 2 bei der Herstellung von optisch aktiven Verbindungen.

4. Verfahren zur Herstellung von optisch aktiven Verbindungen, dadurch gekennzeichnet, daß mindestens ein Teilschritt ein Verfahren nach Anspruch 1 oder 2 beinhaltet.

## Claims

1. A process for preparing enantioselectively acylated alcohols from racemic alcohols, which comprises reacting a racemic alcohol with a diketene with specific lipase catalysis.

2. A process for preparing optically active alcohols from racemic alcohols, which comprises
a) enantioselectively acylating a racemic alcohol with a diketene with specific lipase catalysis,
b) separating the mixture of optically active alcohol and optically active acylated alcohol and thus obtaining one enantiomer of the alcohol,
c) if desired, obtaining the other enantiomer of the alcohol from the acylated alcohol by ester cleavage.

3. The use of a process as claimed in claim 1 or 2 in the preparation of optically active compounds.

4. A process for preparing optically active compounds, wherein at least one step comprises a process as claimed in claim 1 or 2.

## Revendications

1. Procédé pour la préparation d'alcools acylés à acylation énantiosélective à partir d'alcools racémiques, caractérisé par le fait que l'on fait réagir un alcool racémique avec un dicétène avec catalyse spécifique par une lipase.

2. Procédé pour la préparation d'alcools possédant l'activité optique à partir d'alcools racémiques, caractérisé par le fait que
a) on soumet un alcool racémique à acylation énantiosélective par un dicétène avec catalyse spécifique par une lipase,
b) on sépare le mélange de l'alcool possédant l'activité optique et de l'alcool acylé possédant l'activité optique, ce qui permet d'obtenir un énantiomère de l'alcool,
c) si on le désire, on obtient à partir de l'alcool acylé l'autre énantiomère de l'alcool par scission de l'ester.

3. Utilisation d'un procédé selon la revendication 1 ou 2 pour la préparation de composés possédant l'activité optique.

4. Procédé pour la préparation de composés possédant l'activité optique, caractérisé par le fait que l'on réalise au moins un stade opératoire d'un procédé selon la revendication 1 ou 2.
